# EUROPEAN PATENT SPECIFICATION

(11) **EP 2 190 491 B1**
(45) Date of publication and mention of the grant of the patent: **08.08.2012**
(21) Application number: 08827976.5
(22) Date of filing: 14.08.2008
(51) Int. Cl.: A61L 27/30, A61L 27/50, A61L 29/10, A61L 29/14, A61L 31/14, A61L 31/08

(54) **PROCESS FOR THE MANUFACTURE OF MEDICAL DEVICES HAVING SOL-GEL DERIVED CERAMIC REGIONS WITH MOLDED SUBMICRON SURFACE FEATURES**
VERFAHREN ZUR HERSTELLUNG VON MEDIZINISCHEN VORRICHTUNGEN MIT KERAMIKBEREICHEN AUS SOL-GEL MIT GEFORMTEN SUBMIKRONOBERFLÄCHENEIGENSCHAFTEN
PROCÉDÉ POUR FABRIQUER DES DISPOSITIFS MÉDICAUX COMPORTANT DES RÉGIONS EN CÉRAMIQUE DÉRIVÉES DE SOL-GEL À CARACTÉRISTIQUES DE SURFACE MOULÉE DE L'ORDRE DU SOUS-MICRON

(30) Priority: 17.08.2007 US 893849
(43) Date of publication of application: 02.06.2010
(73) Proprietor: Boston Scientific Scimed, Inc., Maple Grove, MN 55311-1566 (US)
(72) Inventor: WEBER, Jan, 6228 GJ Maastricht (NL); ATANASOSKA, Liliana, Edina, MN 55436 (US); ZOROMSKI, Michele, Minneapolis, MN 54406 (US); WARNER, Robert, W., Woodbury, MN 55125 (US)
(74) Representative: Peterreins, Frank
(86) International application number: PCT/US2008/073109
(87) International publication number: WO 2009/026086

(56) References cited:
- EP-A1- 1 891 988
- WO-A1-2005/082277
- US-A1- 2006 199 876
- US-A1- 2007 110 888
- US-B1- 6 709 379
- YIM E K F ET AL: "Significance of synthetic nanostructures in dictating cellular response" NANOMEDICINE: NANOTECHNOLOGY, BIOLOGY AND MEDICINE, ELSEVIER, NL, vol. 1, no. 1, 1 March 2005 (2005-03-01), pages 10-21, XP025360155 ISSN: 1549-9634 [retrieved on 2005-03-01] cited in the application
- CHIATZUN GOH ET AL: "Nanostructuring Titania by Embossing with Polymer Molds Made from Anodic Alumina Templates", NANO LETTERS, ACS, US, vol. 5, no. 8, 1 January 2005 (2005-01-01) , pages 1545-1549, XP007916764, ISSN: 1530-6984, DOI: 10.1021/NL050704C [retrieved on 2005-07-26]

## Description

### TECHNICAL FIELD

The present invention is directed to a process for providing medical devices having featured surfaces, and more particularly to medical devices having sol-gel derived ceramic regions with molded submicron surface features.

### BACKGROUND

It is known that certain ceramic materials are bioactive. As defined herein "bioactive material" is a material that promotes good adhesion with adjacent tissue, for example, bone tissue or soft tissue, with minimal adverse biological effects (e.g., the formation of connective tissue such as fibrous connective tissue). Examples of bioactive ceramic materials, sometimes referred to as "bioceramics," include calcium phosphate ceramics, for example, hydroxyapatite; calcium-phosphate glasses, sometimes referred to as glass ceramics, for example, bioglass; and various metal oxide ceramics, such as titanium oxide, iridium oxide, zirconium oxide, tantalum oxide and niobium oxide, among other materials, in various forms such as rutile, anatase, and perovskite, among others. In this regard, it has been proposed that the formation of bone-like apatite on artificial materials is induced by functional groups, including Si-OH, Ti-OH, Zr-OH, Nb-OH and Ta-OH, among others. T. Kokubo et al., "Novel bioactive materials with different mechanical properties," Biomaterials, 2003, 24(13): 2161-75.

Moreover, it is also known that bioactivity depends upon the structure of a given surface. See, e.g., the review by E.K.F Yim et al., "Significance of synthetic nanostructures in dictating cellular response," Nanomedicine: Nanotechnology, Biology, and Medicine 1 (2005) 10― 21, which reports that smooth muscle cells and endothelial cells have improved cell adhesion and proliferation on nanopatterned surfaces. Both types of cells were sensitive to nanotopography. Yim et al. report improved adhesion and growth for endothelial cells on a substrate with 13 nm high islands relative to 35 and 95 nm high islands. Endothelial cells were also susceptible to surface chemistry. See also, e.g., Viitala R. et al., "Surface properties of in vitro bioactive and non-bioactive sol-gel derived materials," Biomaterials. 2002 Aug; 23(15): 3073-86.

Nanoporous aluminum oxide coatings have been formed on stent platforms using anodization techniques and physical vapor deposition techniques. See, e.g., U.S. Pat. No. 6,709,379 entitled "Implant with cavities containing therapeutic agents," and H. Wieneke et al., Catheterization and Cardiovascular Interventions 60 (2003) 399―407.

WO2005/082277 **describes** implantable medical devices coated with a sol-gel derived ceramic. A coated sacrificial template layer is used to provide the coating with submicron surface features, preferably in the range of 1-100 nm.

US2007/0110888 **relates to** stents with a polymeric drug-eluting coating which is imprinted by molding or embossing techniques by pressing a mold into the coating before the coating is fully dry.

### SUMMARY OF THE INVENTION

According to an aspect of the present invention, a process for providing implantable or insertable medical devices, which contain sol-gel derived ceramic regions which have molded submicron surface features, is provided and defined in claims 1-16.

An advantage of certain embodiments of the present invention is that submicron surface features may be created for a wide variety of materials in addition to alumina, for example, oxides of titanium, zirconium, iridium, tantalum, niobium, ruthenium, tin, and combinations thereof, among many others.

Another advantage of certain embodiments of the present invention is that medical devices can be provided which have controlled biological interactions.

These and other embodiments and advantages of the present invention will become immediately apparent to those of ordinary skill in the art upon review of the Detailed Description and Claims to follow.

### BRIEF DESCRIPTION OF THE DRAWINGS

Fig. 1 is a schematic, partial cross-sectional view of an assembly, which includes a sol-gel precursor disposed between a planar medical device substrate and a planar mold, in accordance with an embodiment of the present invention.

Fig. 2A is a schematic, cross-sectional view of an assembly, which includes a sol-gel precursor disposed between a tubular medical device substrate and a planar mold, in accordance with an embodiment of the present invention.

Fig. 2B is a schematic, cross-sectional view of an assembly, which includes a sol-gel precursor disposed between a tubular planar medical device substrate and a solid cylindrical mold, in accordance with an embodiment of the present invention.

Fig. 3A and 3B are schematic, cross-sectional views of assemblies, each of which includes a sol-gel precursor disposed between a tubular medical device substrate and a planar mold which has been wrapped into the form of a tube, in accordance with two embodiments of the present invention.

Fig. 4A is a schematic, cross-sectional view of an assembly, which includes a sol-gel precursor disposed between a tubular medical device substrate and a hollow cylindrical mold, in accordance with an embodiment of the present invention.

Fig. 4B is a schematic, cross-sectional view of an assembly, which includes a sol-gel precursor disposed between a tubular medical device substrate and a hollow cylindrical mold, in accordance with another embodiment of the present invention.

Fig. 4C is a schematic, cross-sectional view of an assembly, which includes a sol-gel precursor disposed between a tubular medical device substrate and a hollow cylindrical mold that is reinforced by a reinforcement element, in accordance with another embodiment of the present invention.

Fig. 5 is a schematic, cross-sectional view illustrating an assembly in accordance the present invention, which includes a sol-gel precursor disposed between a hollow cylindrical mold and an additional mold component.

### DETAILED DESCRIPTION

As noted above, in one aspect, the process of present invention provides implantable or insertable medical devices, which contain sol-gel derived ceramic regions that have molded submicron surface features.

As used herein, a "ceramic region" is a region (e.g., monolithic region, a coating layer, etc.) that contains one or more ceramic materials (e.g., one or more metal and/or semi-metal oxides such as those discussed below, among others), for example, containing one or more ceramic materials in an amount ranging from 50 wt% or less to 75 wt% to 90 wt% to 95 wt% to 97.5 wt% to 99 wt% or more.

As used herein, a "sol-gel derived ceramic region" is a ceramic region that is formed using sol-gel chemistry.

As used herein a "submicron surface feature" is a physical feature, for example, a pore, trench, or other depression, or a knob, ridge, or other projection, which has a width that does not exceed one micron (1 µm). In some embodiments, the submicron surface features of the invention are dimensioned and spaced in a way that improves the bioactivity of the ceramic region.

As used herein "molded" submicron surface features are those that have been created using a mold.

As used herein, a "mold" is a template which has features that are inverse to those that are created by the mold, for example, by stamping an impressionable material with the mold or by solidification of a fluid material in the presence of the mold.

As used herein, a "submicron pore" is a pore having a width that does not exceed 1 micron. As used herein, a "nanopore" is a pore having a width that does not exceed 50 nm. As used herein, nanopores include "micropores," which are smaller than 2 nm in width and "mesopores," which range from 2 to 50 nm in width. As used herein, "macropores" are larger than 50 nm in width and are thus not nanopores. As used herein a "porous surface" is a surface that contains pores. A "sub-micro-porous surface" is a surface that contains submicron pores. A "nanoporous surface" is a surface that contains nanopores; a "macroporous surface" is a surface that contains macropores; and so forth.

In some embodiments of the invention, dense porous surfaces are produced. As used herein a "dense porous surface" is one whereby the surface area in between the pores is less than 75% of the total surface area. In some embodiments, the porous surface is one whose pores have an average center-to-center spacing to their nearest neighbors that is less than three times the average pore width.

As noted above, "submicron features" are smaller than 1 micron in width. As used herein a "featured surface" is a surface that contains features such as those described above. A "submicron featured surface" is a surface that contains submicron features. A "nanofeature" is a feature having a width that does not exceed 50 nm. As used herein, nanofeatures include "microfeatures," which are smaller than 2 nm in width and "mesofeatures," which range from 2 to 50 nm in width. As used herein, "macrofeatures" are larger than 50 nm in width and are thus not nanofeatures. A "nanofeatured surface" is a surface that contains nanofeatures; a "macrofeatured surface" is a surface that contains macrofeatures; and so forth.

Medical devices benefiting from the present invention include a variety of implantable or insertable medical devices, which are implanted or inserted into a subject, either for procedural uses or as implants. Examples include stents (including coronary artery stents, peripheral vascular stents such as cerebral stents, urethral stents, ureteral stents, biliary stents, tracheal stents, gastrointestinal stents and esophageal stents), stent grafts, vascular grafts, abdominal aortic aneurysm (AAA) devices (e.g., AAA stents, AAA grafts), vascular access ports, dialysis ports, catheters (e.g., renal or vascular catheters such as balloon catheters), guide wires, balloons, filters (e.g., vena cava filters), vascular access ports, embolization devices including cerebral aneurysm filler coils (including Guglielmi detachable coils and metal coils), microspheres or other particles including embolic particles and tissue bulking particles, septal defect closure devices, drug depots that are adapted for placement in an artery for treatment of the portion of the artery distal to the device, myocardial plugs, patches, pacemakers, leads including pacemaker leads, defibrillation leads, and coils, ventricular assist devices including left ventricular assist hearts and pumps, total artificial hearts, shunts, valves including heart valves and vascular valves, tissue engineering scaffolds for cartilage, bone, skin and other in vivo tissue regeneration, cochlear implants, sutures, suture anchors, anastomosis clips and rings, tissue staples and ligating clips at surgical sites, cannulae, metal wire ligatures, orthopedic prosthesis such as bone grafts, bone plates, fins and fusion devices, joint prostheses, spinal discs and nuclei, as well as various other medical devices that are adapted for implantation or insertion into the body for treatment or diagnosis of various diseases and conditions.

The medical devices of the present invention include implantable and insertable medical devices that are used for systemic treatment or diagnosis, as well as those that are used for the localized treatment or diagnosis of any mammalian tissue or organ. Nonlimiting examples are tumors; organs including the heart, coronary and peripheral vascular system (referred to overall as "the vasculature"), the urogenital system, including kidneys, bladder, urethra, ureters, prostate, vagina, uterus and ovaries, eyes, ears, spine, nervous system, lungs, trachea, esophagus, intestines, stomach, brain, liver and pancreas, skeletal muscle, smooth muscle, breast, dermal tissue, cartilage, tooth and bone.

As used herein, "treatment" refers to the prevention of a disease or condition, the reduction or elimination of symptoms associated with a disease or condition, or the substantial or complete elimination of a disease or condition. Preferred subjects (also referred to as "patients") are vertebrate subjects, more preferably mammalian subjects and more preferably human subjects. Specific examples of medical devices for use in conjunction with the present invention include vascular stents, such as coronary stents and cerebral stents, which deliver a therapeutic agent into the vasculature for the treatment of restenosis.

In some embodiments, the sol-gel derived ceramic regions of the present invention correspond to an entire medical device. In other embodiments, the sol-gel derived ceramic regions correspond or to one or more portions of a medical device. For instance, the sol-gel derived ceramic regions can be in the form of one or more discrete medical device components, in the form of one or more sol-gel derived ceramic layers disposed over all or only a portion of an underlying medical device substrate, and so forth. Layers can be provided over an underlying substrate at a variety of locations, and in a variety of shapes (e.g., in desired patterns, for instance, using appropriate application or masking techniques), and they can be of different compositions. As used herein a "layer" of a given material is a region of that material whose thickness is small compared to both its length and width. As used herein a layer need not be planar, for example, taking on the contours of an underlying substrate. Layers can be discontinuous (e.g., patterned). Terms such as "film," "layer" and "coating" may be used interchangeably herein.

Materials for use as underlying substrates include polymeric materials, ceramic materials and metallic materials.

Specific examples of polymeric materials may be selected, for example, from fluoropolymers such as polytetrafluoroethylene (PTFE), various polyvinyl polymers, and various polyurethanes, polymers that can be dissolved preferential relative to sol-gel derived ceramic regions, such as polymethylmethacrylate (PMMA), among many others.

Specific examples of ceramic substrate materials may be selected, for example, from materials containing one or more of the following: metal oxides, including aluminum oxides and transition metal oxides (e.g., oxides of titanium, zirconium, ruthenium, niobium, hafnium, tantalum, molybdenum, tungsten, rhenium, and iridium); silicon; silicon-based ceramics, such as those containing silicon nitrides, silicon carbides and silicon oxides (sometimes referred to as glass ceramics); calcium phosphate ceramics (e.g., hydroxyapatite); carbon and carbon-based, ceramic-like materials such as carbon nitrides, among many others.

Specific examples of metallic substrate materials may be selected, for example, from materials containing one or more of the following: substantially pure metals, including gold, platinum, palladium, iridium, osmium, rhodium, titanium, zirconium, tantalum, tungsten, niobium, and ruthenium, and metal alloys, including metal alloys comprising iron and chromium (e.g., stainless steels, including platinum-enriched radiopaque stainless steel), niobium alloys, titanium alloys, nickel alloys including alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N), alloys comprising cobalt, chromium, tungsten and nickel (e.g., L605), and alloys comprising nickel and chromium (e.g., inconel alloys), among many others.

Further examples of metallic substrate materials include the biodegradable metallic materials described in U.S. Patent App. Pub. No. 2002/0004060 A1, entitled "Metallic implant which is degradable in vivo." These include substantially pure metals and metal alloys whose main constituent is selected from alkali metals, alkaline earth metals, iron, and zinc, for example, metals and metal alloys containing magnesium, iron or zinc as a main constituent and one or more additional constituents selected from the following: alkali metals such as Li, alkaline-earth metals such as Ca and Mg, transition metals such as Mn, Co, Ni, Cr, Cu, Cd, Zr, Ag, Au, Pd, Pt, Re, Fe and Zn, Group IIIa metals such as Al, and Group IVa elements such as C, Si, Sn and Pb.

Sol-gel derived ceramic regions in accordance with the present invention may be formed from various ceramic materials, including various metal-oxides, semi-metal-oxides and combinations thereof. For example, sol-gel derived ceramic regions in accordance with the present invention may be formed from oxides of silicon, germanium, aluminum, zirconium, titanium, tin, iron, hafnium, niobium, tantalum, molybdenum, tungsten, rhenium and iridium, as well as combinations of oxides of two or more of the preceding metals and semi-metals.

In a typical sol-gel process, precursor materials, typically selected from inorganic metallic and semi-metallic salts, metallic and semi-metallic complexes/chelates, metallic and semi-metallic hydroxides, and organometallic and organo-semi-metallic compounds such as metal alkoxides and alkoxysilanes, are subjected to hydrolysis and condensation (also referred to sometimes as polymerization) reactions, thereby forming a "sol" (i.e., a suspension of solid particles within a liquid).

For example, an alkoxide of choice (such as a methoxide, ethoxide, isopropoxide, *tert*-butoxide, etc.) of a semi-metal or metal of choice (such as silicon, germanium, aluminum, zirconium, titanium, tin, iron, hafnium, niobium, tantalum, molybdenum, tungsten, rhenium, iridium, etc.) may be dissolved in a suitable solvent, for example, in one or more alcohols. Subsequently, water or another aqueous solution, such as an acidic or basic aqueous solution (which aqueous solution can further contain organic solvent species such as alcohols) is added, causing hydrolysis and condensation to occur. If desired, additional agents can be added, such as agents to control the viscosity and/or surface tension of the sol, among others.

The sol-gel reaction is basically understood to be a ceramic network forming process as illustrated in the following simplified scheme from G. Kickelbick, "Prog. Polym. Sci., 28 (2003) 83-114: 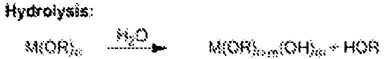 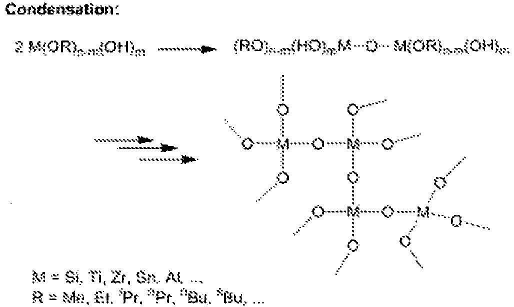 in which the metal/semi-metal atoms (designated generally herein as M) within the ceramic phases are shown to be linked to one another via covalent linkages, such as M— 0—M linkages, although with other interactions that are also commonly present including, for example, hydrogen bonding due to the presence of hydroxyl groups such as residual M―OH groups within the network. As noted above, it has been proposed that the formation of bone-like apatite on artificial materials is induced by functional groups, including Si-OH, Ti-OH, Zr-OH, Nb-OH and Ta-OH, among others,

In a typical sol-gel process a so-called "wet gel" is formed from the sol (e.g., by coating a sol on a substrate). The wet gel is then dried. If the solvent in the wet gel is removed under supercritical conditions, a material commonly called an "aerogel" is obtained. If the gel is dried via freeze drying (lyophilization), the resulting material is commonly referred to as a "cryogel." Drying at ambient temperature and ambient pressure leads to what is commonly referred to as a "xerogel." Other drying possibilities are available including elevated temperature drying (e.g., in an oven), vacuum drying (e.g., at ambient or elevated temperatures), and so forth. Further information concerning sol-gel materials can be found, for example, in Viitala R. et al., "Surface properties of in vitro bioactive and non-bioactive sol-gel derived materials," Biomaterials, 2002 Aug; 23(15):3073-86.

The use of sol-gel processing in the formation of surfaces with sub-micron pores has been demonstrated. In particular, C. Goh et al., Nano Lett., Vol. 5, No. 8, 2005, 1545-1549, have recently exposed a titania sol-gel precursor to poly(methyl methacrylate) (PMMA) molds to make thin films of titania having dense arrays of 35-65 nm diameter pores.

The process begins by preparing a template from which a polymeric mold can be formed. Metal and metal oxide molds are useful, because, as seen further below, they can be dissolved under conditions which do no substantially affect the polymeric mold.

Anodic alumina templates are particularly appealing for this purpose, because the aluminum anodization process is extremely robust, accurate and reproducible. The anodization conditions for making porous anodic alumina are well documented in the literature, with pore spacing and pore diameter being tuned by using different acidic baths and by adjusting the anodization voltages, and pore depth (which generally corresponds to the thickness of the anodized aluminum layer) being tuned by adjusting the anodization time. By varying such parameters, pore sizes ranging, for example, from 5 to 420 nm have been reported. The individual pores that are formed in the alumina upon anodization process may be random or they may be ordered, for example, in a hexagonally packed structure. Pore ordering has been shown to be improved using highpurity aluminum films, which are preannealed and electropolished. Pore ordering also depends on anodization conditions, including the anodization voltage and the selected electrolyte. Pore ordering may be promoted through the use of a pre-texturing process in which an array of shallow concave features is initially formed on aluminum by indentation. Pore ordering may also be promoted by employing a two-step anodization method. The first step involves anodization of high purity aluminum to form a porous alumina layer. This layer is then dissolved, yielding a patterned aluminum substrate with an ordered array of concave features formed during the first anodization step. The ordered concave features then serve as the initial sites to form a highly ordered nanopore array in a second anodization step. Aluminum anodization normally results in a porous alumina structure which is separated from the aluminum substrate by a layer of Al₂O₃. For further information on anodic alumina processing, see, e.g., H.X. He et al., "Electrochemical fabrication of metal nanowires" in Encyclopedia of Nanoscience and Nanotechnology, Ed., N.S. Nalwa, American Scientific Publishers, 2003, F. Li et al. Chem. Mater. 1998, 10, 2470-2480 and A.P. Li et al, J. Appl. Phys., 1998, 84(11), 6023-6026, as well as the references cited therein.

In addition to selective etching processes, molds may also be formed using selective deposition processes and selective milling processes, among others. For example, molds may be provided with sub-micron features using a process known as FIB (focused ion beam sputtering and deposition). Such technology is quite advanced at the present time as seen, for example, from T. Tanaka et al., Thin Solid Films 509 (2006) 113―117, and the references cited therein. Firms specializing in this technology include Fibics Inc., Ottawa, Canada.

Once a template having suitable surface features is obtained, a polymer can be introduced to the template (e.g., by infiltrating the polymer into the pores of the template, etc.). For example, a suitable monomer may be polymerized in the presence of the template, or a polymer in fluid form (e.g., in the form of a melt, solution, and/or uncured polymeric precursor) may be introduced to the template (e.g., by spin coating, spray coating, dip coating, ink jet printing, coating with an applicator such as a roller brush or blade, etc.) and solidified.

For example, in Goh et al. *supra,* molds were prepared by spin coating a thin layer PMMA in chlorobenzene solution onto an anodic aluminum template, and subsequently heating the sample to 200 °C to assist infiltration into the pores of the template. PMMA is a desirable replicating material for several reasons including the following: (a) it is a relatively high modulus polymer, allowing dense and small features to be replicated, (b) it can be heated to assist in infiltrating the template, (b) it can be dissolved in organic solvents for easy application to templates and easy removal from sol-gel derived ceramic regions, and (d) it is stable in aqueous and/or alcoholic solutions, which are used in the process of separating the PMMA from the template and in sol-gel processing. A 1-mmthick layer of polydimethylsiloxane (PDMS), a much softer material, was coated over the PMMA in Goh et al. and cured at room temperature to provide a backing layer for the mold, in order to provide ease of manipulation and to provide the mold with sufficient flexibility to conform well to various substrates. The mold is then removed from the template, for example, by pulling the mold away from the template or destroying the template. For example, a PDMS/PMMA mold may be removed from an anodized alumina template by first wet etching the aluminum portion of the template in FeCl₃/HCl, followed by wet etching the alumina portion in NaOH. *Id.*

Once a suitable mold is obtained it can be used in conjunction with sol-gel processing to form a porous region of desired shape and size. For example, in Goh et al. *supra,* a sol-gel precursor (i.e., titanium ethoxide, HCl and 2-propanol) was spin coated onto a substrate followed by contact with a mold before the precursor became dried. Because this method led to a high density of voids on the substrate, the sol-gel precursor solution was also spin coated on the mold, followed by contact with the substrate prior to precursor drying. After the precursor dried, the PDMS was peeled off. The remaining PMMA, which was strongly adhered to the resultant Ti0₂, was then dissolved in acetonitrile. The resulting porous Ti0₂ film was finally calcined in air, yielding thin films of titania having dense arrays of 35-65 nm diameter pores.

In accordance with the present invention, medical devices having submicron surface features are formed using these techniques. Unlike the sol-gel derived titanium oxide layers described in Goh et al., which were deposited on silicon wafers, indium-tin oxide coated glass substrates and fluorine doped tin oxide coated glass substrates (and which were employed for their use in photovoltaic and photocatalyic applications), the sol-gel derived ceramic regions of the present invention are provided for medical applications, typically for medical applications requiring biocompatibility (including bioactivity) and/or drug delivery applications.

Furthermore, although nanoporous titania ceramic materials are formed in Goh et al., devices having sol-gel derived ceramic materials other than titania are within the scope of the present invention, including those metal and semi-metal oxides specifically listed above, among others. In addition, features other than nanopores are within the scope of the present invention, including submicron trenches and other submicron depressions, submicron knobs, submicron ridges and other submicron projections, as well as combinations of the same.

For example, it is known that submicron features are able to stimulate or slow cell growth or proliferation. See, e.g., E.K.F. Yim et al., Nanomedicine: Nanotechnology, Biology, and Medicine 1 (2005) 10― 21 and S. Buttiglieri et al., Biomaterials 24 (2003) 2731-2738. For example, it has been shown that smooth muscle cell proliferation can be reduced by ordered patterns (e.g., 350 nm lines) whereas endothelial cells are stimulated (e.g., by 13 nm hills). In certain embodiments of the invention, submicron lines (i.e., linear features in the form or ridges or trenches having a width of about 200 to 500 nm (and having the same depth or spacing) are formed to inhibit smooth muscle cell proliferation. If desired lines of these dimensions may be provided with 13 nm protrusions (e.g., nanodots, nanoknobs, nanodomes, etc.). In the case of a vascular stent, lines may be provided on the struts of the stent, which are parallel to the longitudinal axis of the stent once the stent is deployed. Where the stent is deformed (e.g., bent) during the course of deployment, features may be created to take this into account. For example, because the geometry of the stent is known both before and after deployment, one can calculate the change in orientation for each stent element from the pre-deployed stage (e.g., after laser cutting) to the post-deployed stage, and take this mapping into account in making the mold.

Moreover, ceramic regions in accordance with the present invention may be formed, which further include one or more polymers, based upon sol-gel processes, as well as upon principles of polymer synthesis, manipulation and processing. Sol-gel processes are suitable for use in conjunction with polymers and their precursors, for example, because they can be performed at ambient temperatures. A review of various techniques for generating polymeric-ceramic composites can be found, for example, in G. Kickelbick, Prog. Polym. Sci., 28 (2003) 83-114.

For example, polymer-containing ceramic regions may be formed by impregnating a gel such as a xerogel with a monomer and polymerizing the monomer within the gel. Enhanced results may be obtained with techniques of this type, where interactions between the monomer/polymer and the gel are sufficiently strong to prevent macroscopic phase separation.

Conversely, polymer-containing ceramic regions may be formed, for example, by including a preformed polymer within the sol-gel precursor. As above, enhanced results may be obtained where interactions between the polymeric and the ceramic components are sufficiently strong to prevent macroscopic phase separation.

Polymer-containing ceramic regions with submicron phase domains may be created by providing covalent interactions between the polymeric and ceramic components, for example, through one of the following: (a) providing a sol-gel precursor that includes polymers with ceramic precursor groups (e.g., groups that are capable of participation in hydrolysis/condensation, such as metal or semi-metal alkoxide groups), (b) providing a sol-gel precursor that includes both ceramic precursor groups and polymer precursor groups and thereafter proceeding with hydrolysis/condensation and polymerization reactions, either simultaneously or sequentially, (c) forming a ceramic region which contains polymer precursor groups (e.g., groups that are capable of participation in a polymerization reaction, such as vinyl groups or cyclic ether groups) and thereafter conducting one or more polymerization steps, and so forth.

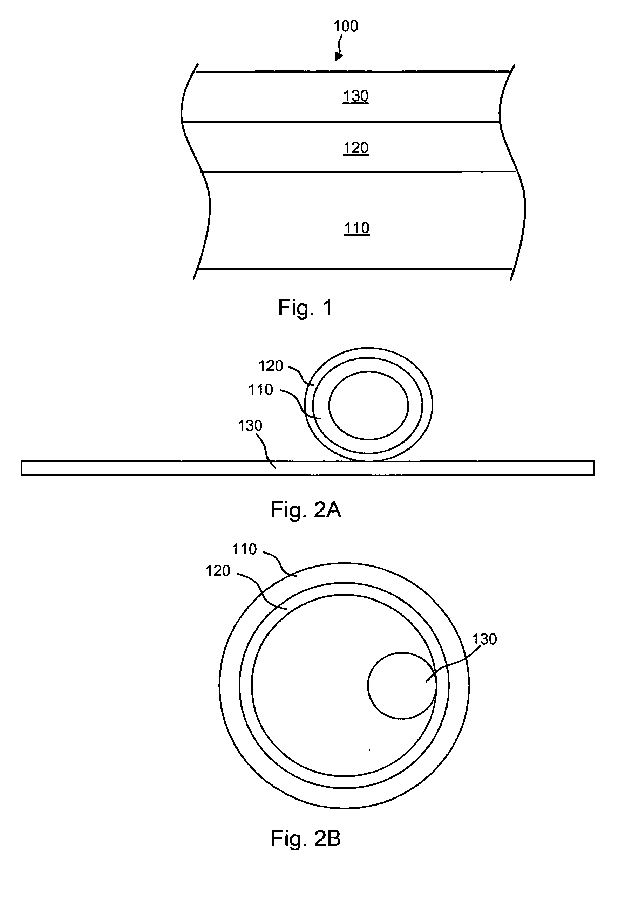

Turning now to Fig. 1 a partial schematic view of an assembly 100 in accordance with the present invention is illustrated, which includes a sol-gel precursor 120 disposed between a planar medical device substrate 110 and a planar mold 130. The sol-gel precursor 120 may be applied to the mold 130, for example, by spin coating as described above, followed immediately by application to the substrate 110. After the sol-gel precursor 120 has dried, the mold 130 may be removed, for example, by solvent dissolution as described above, or another method, if practical, including physical detachment of the mold or mold destruction during a subsequent heating step.

In various other embodiments of the invention, however, sol-gel derived ceramic regions are formed which are not planar. In certain of these embodiments, a planar mold, for example one based on an anodized aluminum template or a template formed using focused ion beam sputtering and/or deposition, among other techniques, may be used to construct a non-planar sol-gel derived region.

For example, in accordance with an embodiment of the invention illustrated in schematic cross-section in Fig. 2A, a rotating concept may be employed in which a tubular medical device 110 having a sol-gel precursor 120 on its outer surface is rolled against a planar mold 130, forming submicron features in the sol-gel precursor 120 that correspond inversely to projections/depressions on the surface of the mold 130. After the sol-gel precursor 120 has dried, it may be heated, for example, if desired for calcination.

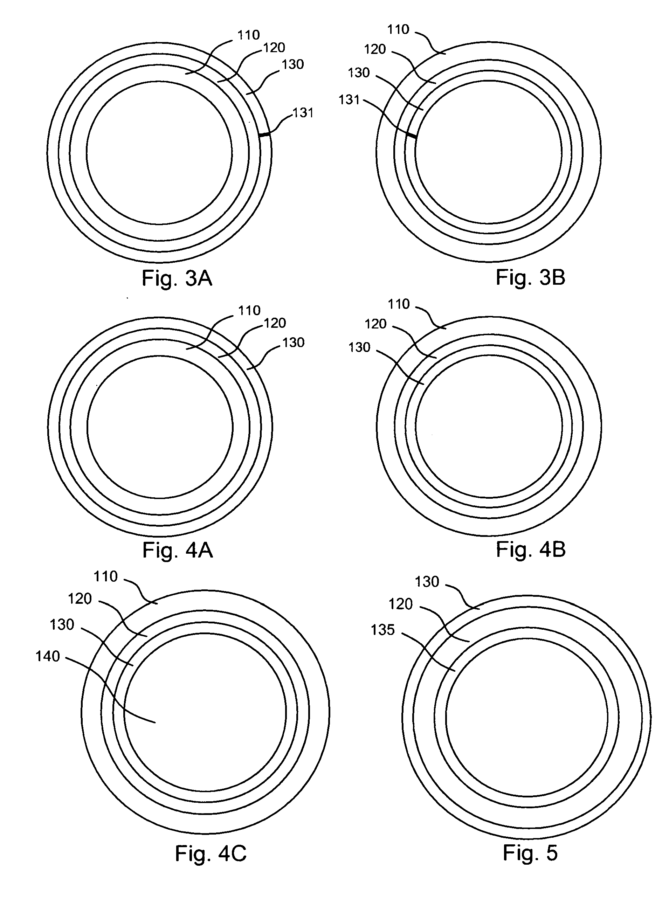

As another example, Fig. 3A is a schematic cross-sectional view illustrating an assembly in accordance with an embodiment of the invention, which includes a sol-gel precursor 120 disposed between a tubular medical device substrate 110 and a mold 130. The sol-gel precursor 120 may be applied to the mold 130, for example, by spin coating as described above, followed immediate application to the outside surface of the substrate 110, for instance, by wrapping the sol-gel-precursor-coated mold around the substrate. The location 131 where the ends of the mold 130 meet upon wrapping is also shown. After the sol-gel precursor 120 has dried, the mold 130 may be removed, for example, as described above, and the resulting device may be heated, as desired.

Like Fig. 3A, Fig. 3B is a partial schematic cross-sectional view illustrating an assembly in accordance the present invention, which includes a sol-gel precursor 120 disposed between a tubular medical device substrate 110 and a mold 130. In Fig. 3B, however, the mold 130 is positioned on the inside of the substrate 110, rather than the outside. The location 131 where the ends of the mold 130 meet upon wrapping is also shown in Fig. 3B. After the sol-gel precursor 120 has dried, the mold 130 may be removed, for example, as described above, and the resulting device may be heated, as desired.

In other embodiments of the invention, a non-planar template, for example, a non-planar anodized alumina template or a template formed using focused ion beam sputtering and/or deposition, among other techniques, is used for mold creation. For example, a hollow cylindrical aluminum form may be anodized at its inner surface, or a hollow or solid cylindrical aluminum form may be anodized at its outer surface. For this purpose, a non-planar counter electrode may be used, for example, one which has a suitable geometric configuration to take into account current distribution effects. For example, a counter electrode having a hollow cylindrical geometry may be disposed outside of and coaxial with a hollow or solid cylindrical aluminum form, or a counter electrode having a hollow or solid cylindrical geometry may be disposed inside and coaxial with a hollow cylindrical aluminum form. As another example, a hollow or solid cylindrical aluminum form may be milled at its outer surface using focused ion beam sputtering. As another example, surface features may be formed on the outer surface of a hollow or solid cylindrical aluminum form using focused ion beam deposition.

Using such templates in combination with techniques analogous to those described above, hollow cylindrical molds may be created in which projections are created on the inner surface or hollow or solid cylindrical molds may be created in which projections are created on the outer surface. These can then be used to create submicron surface features in sol-gel derived ceramic regions.

For example, in accordance with an embodiment of the invention illustrated in schematic cross-section in Fig. 2B, a rotating concept may be employed by a method analogous to that illustrated in Fig. 2A. However, in Fig. 2B, the tubular medical device 110 has a sol-gel precursor 120 on its hollow inner surface. Moreover, a cylindrical mold 130 (e.g., in the shape of a rod) is rolled around the inner circumference of the sol-gel-precursor-coated medical device to form submicron features in the sol-gel precursor 120. Alternatively, medical device 110 with sol-gel precursor 120 coating may be rotated around the cylindrical mold 130 to form submicron features in the sol-gel precursor 120. In either case, after the sol-gel precursor 120 has dried, the resulting device may be heated, as desired.

As another example, Fig. 4A is a schematic cross-sectional view illustrating an assembly in accordance with an embodiment of the invention, which includes a sol-gel precursor 120 disposed between a tubular medical device substrate 110 and a hollow cylindrical mold 130. For instance, the sol-gel precursor 120 may be applied to the inner surface of the mold 130 by a suitable technique, followed by immediate contact with the substrate 110. In one particular embodiment, the substrate 110 is a balloon-expandable stent, which may be expanded within the mold 130 for enhanced engagement between the substrate 110 and the sol-gel precursor 120 on the mold 130. After the sol-gel precursor 120 has dried, the mold 130 may be removed, for example, as described above, and the resulting device may be heated, as desired.

Fig. 4B is a schematic cross-sectional view illustrating an assembly in accordance the present invention, which includes a sol-gel precursor 120 disposed between a tubular medical device substrate 110 and a hollow cylindrical mold 130 (which mold may also be solid, if desired). In contrast to Fig. 4A, the mold 130 in Fig. 4B is positioned on the inside of the substrate 110. For instance, the sol-gel precursor 120 may be applied to the outside surface of the mold 130 by a suitable technique, followed by immediate contact with the substrate 110. In one particular embodiment, the substrate 110 is a balloon-expandable stent, which may be positioned over the mold while in an expanded state and then compressed onto the outer surface of the mold 130 for enhanced engagement between the substrate 110 and the sol-gel precursor 120 on the mold 130.

If desired, the mold 130 may be reinforced by a reinforcement element 140 as shown in Fig. 4C. (In embodiments where the mold is in the form of a solid cylinder, such reinforcement is, of course, inapplicable.) Reinforcement element 140 may be, for example, a rod or an apparatus that is expandable within the mold. In the latter case, and in the instance where the mold 130 has sufficient elasticity, the expandable reinforcement element 140 may be used to expand the mold 130 for better engagement between the sol-gel precursor 120 on the mold 130 and the substrate 110.

Regardless of the embodiment selected, after the sol-gel precursor 120 has dried, the mold 130 may be removed, for example, as describe above, and may be heated, as desired.

In other embodiments of the invention, a mold can be formed as described above and used to generate a monolithic sol-gel derived region. For example, Fig. 5 is a schematic cross-sectional view illustrating an assembly in accordance the present invention, which includes a sol-gel precursor 120 disposed between a hollow cylindrical mold 130 prepared, for example, using a cylindrical template prepared as described above, and an additional mold component 135, which may or may not be prepared as described above (e.g., the additional mold component may or may not be provided with submicron surface features). The space between the mold 130 and additional mold component 135 is filled with a sol-gel precursor 120. After the sol-gel precursor 120 has dried, the mold 130 may be removed, for example, as described above, to yield a porous monolithic sol-gel derived region, which may then be heated, as desired.

As a final example of a method for forming a stent, a sol-gel derived ceramic layer having molded submicron surface features according to the invention is first created on the abluminal surface of a metallic tube. Then, the tube is cut into a stent, for example, by means of a femto-second ablating laser. The resulting stent in this embodiment has a ceramic layer on its abluminal surface, which may be useful, for example, in case of preferential abluminal drug delivery. Because the tube is stable in shape compared to an already formed stent, the preceding process is advantageous, for example, in that it allows parts of the deposited sol-gel layer to be selectively removed with the ablating laser without affecting the integrity of the underlying metal structure.

In a specific example, a sol-gel derived ceramic layer of very homogeneous thickness can be deposited on the abluminal surface of a metallic tube, after which the tube is mounted into a laser ablation apparatus (e.g., one which allows tube rotation and axial movement of the tube underneath the laser beam with nanometer precision), allowing the selective removal of the sol-gel layer in those areas corresponding to the high strain areas of the intended stent pattern. The sol-gel layer is removed without cutting (ablating) the stent in this step. After this has been done, the metal can be cut (ablated) to produce the stent pattern.

In addition, because the tube can be remounted onto the laser cutting apparatus at exactly the same position (e.g., by using a simple slot on the tube), one can first ablate reservoirs (e.g., little pockets) in the tube in positions corresponding to the struts to be formed, remove the tube, deposit PMMA into the pockets, polish the tube to make the PMMA in the pockets flush with the surface, provide a sol-gel layer, and mold the sol-gel. If the sol-gel layer is not porous enough to allow the subsequently applied solvent (see below) to pass through, then the molding process can be used to create holes in the sol-gel, for instance, by ensuring that the pattern on the template can reach the PMMA layer. The PMMA is then removed (e.g., with an acetonitrile-containing solvent). PMMA dissolves cleanly, and at a constant rate, in a 7:3 mixture of 1-butanol and acetonitrile. Mixtures of other alcohols (e.g., methanol, ethanol, 2-propanol, hexanol, etc.) with acetonitrile also dissolve PMMA at varying rates. The stent pattern is then cut. As a result of this process, one can create hollow pockets underneath a porous ceramic membrane.

Using techniques such as those described above, for example, a stent may be formed that comprises molded submicron surface features on its inner surface (luminal surface), its outer surface (abluminal surface), or both.

Although planar and tubular medical devices are described in the specific embodiments above, it will be clear to those of ordinary skill in the art that devices having other shapes are within the scope of the present invention.

The medical devices of the present invention also optionally contain one or more therapeutic agents. "Therapeutic agents," "drugs," "pharmaceutically active agents," "pharmaceutically active materials," and other related terms may be used interchangeably herein. These terms include genetic therapeutic agents, non-genetic therapeutic agents, and cells.

Exemplary non-genetic therapeutic agents for use in conjunction with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sutfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) alpha receptor antagonist (such as doxazosin, Tamsulosin) and beta receptor agonists (such as dobutamine, salmeterol), beta receptor antagonist (such as atenolol, metaprolol, butoxamine), angiotensin-II receptor antagonists (such as losartan, valsartan, irbesartan, candesartan and telmisartan), and antispasmodic drugs (such as oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.), (z) selective estrogen receptor modulators (SERMs) such as raloxifene, lasofoxifene, arzoxifene, miproxifene, ospemifene, PKS 3741, MF 101 and SR 16234, (aa) PPAR agonists such as rosiglitazone, pioglitazone, netoglitazone, fenofibrate, bexaotene, metaglidasen, rivoglitazone and tesaglitazar, (bb) prostaglandin E agonists such as alprostadil or ONO 8815Ly, (cc) thrombin receptor activating peptide (TRAP), (dd) vasopeptidase inhibitors including benazepril, fosinopril, lisinopril, quinapril, ramipril, imidapril, delapril, moexipril and spirapril, (ee) thymosin beta 4.

Specific examples of non-genetic therapeutic agents include taxanes such as paclitaxel, (including particulate forms thereof, for instance, protein-bound paclitaxel particles such as albumin-bound paclitaxel nanoparticles, e.g., ABRAXANE), sirolimus, everolimus, tacrolimus, zotarolimus, Epo D, dexamethasone, estradiol, halofuginone, cilostazole, geldanamycin, alagebrium chloride (ALT-711), ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2) , as well a derivatives of the forgoing, among others.

Exemplary genetic therapeutic agents for use in conjunction with the present invention include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in conjunction with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis (i.e., antirestentotic agents). Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, such as bosentan, sitaxsentan sodium, atrasentan, endonentan, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomer), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE 1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, atorvastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD (orgotein), SOD mimics, verteporfin, rostaporfin, AGI 1067, and M 40419, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) matrix metalloprotease (MMP) pathway inhibitors such as marimastat, ilomastat, metastat, pentosan polysulfate, rebimastat, incyclinide, apratastat, PG 116800, RO 1130830 or ABT 518, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), olimus family drugs (e.g., sirolimus, everolimus, tacrolimus, zotarolimus, etc.), cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, (cc) blood rheology modulators such as pentoxifylline and (dd) gluclose cross-link breakers such as alagebrium chloride (ALT-711).

Further additional therapeutic agents useful for the practice of the present invention are also disclosed in U.S. Patent No. 5,733,925 to Kunz.

Where a therapeutic agent is included, a wide range of therapeutic agent loadings can be used in conjunction with the medical devices of the present invention, with the therapeutically effective amount being readily determined by those of ordinary skill in the art and ultimately depending, for example, upon the condition to be treated, the age, sex and condition of the patient, the nature of the therapeutic agent, the nature of the ceramic region(s), and/or the nature of the medical device, among other factors.

Therapeutic agents and/or other optional additives may be introduced subsequent to the formation of the sol-gel derived ceramic region in some embodiments. This may be suitable, for example, where the sol-gel is subjected to high temperatures, for example, to temperatures of 100°C, 200°C, 300°C, 400°C, 500°C, or more. Such high temperatures commonly reduce the porosity of the sol-gel, while at the same time increasing its mechanical strength. For instance, in some embodiments, the therapeutic agent and/or other optional additives are dissolved or dispersed within a solvent, and the resulting solution contacted with a previously formed ceramic region (e.g., using one or more of the application techniques described above, such as dipping, spraying, etc.) to load the ceramic region with the therapeutic agent.

In other embodiments, sol-gel processing may be carried out at low temperatures (e.g., temperatures of 50°C or less). This aspect of the present invention permits the incorporation of temperature sensitive therapeutic agent during the course sol-gel processing.

In still other embodiments, the sol-gel derived ceramic material may be formed and then disposed over a therapeutic agent containing region on the medical device surface (e.g., by adhesion).

## Claims

1. A process for providing an implantable or insertable medical device (100) comprising a sol-gel derived ceramic region that comprises molded submicron surface features, including the steps of:
applying a sol-gel precursor (120) to a mold (130) or a medical device substrate (110);
applying the mold (130) to the medical device substrate (110) after applying the sol-gel precursor (120); and
removing the mold (130) after the sol-gel precursor (120) has dried.

2. The process of claim 1, wherein the medical device (100) is a tubular medical device and wherein:
the sol-gel precursor (120) is applied to the mold (130); and
applying the mold (130) includes wrapping the sol-gel precursor-coated mold (130) around the substrate (110).

3. The process of claim 1, wherein the medical device (100) is a tubular medical device and wherein:
the sol-gel precursor (120) is applied to the mold (130); and
applying the mold (130) includes wrapping the sol-gel precursor-coated mold (130) around the substrate (110), wherein the mold (130) is positioned on the inside of the substrate (110).

4. The process of claim 1, wherein the medical device (100) is a tubular medical device and the mold (130) is a cylindrical mold (130), wherein
the sol-gel precursor (120) is applied to an hollow inner surface of the medical device; and
the cylindrical mold (130) is rolled around an inner circumference of the sol-gel precursor-coated medical device to form the submicron features.

5. The process of claim 1, wherein the medical device (100) is a tubular medical device and the mold (130) is a cylindrical mold (130), wherein
the sol-gel precursor (120) is applied to an hollow inner surface of the medical device; and
the medical device substrate (110) is rolled around the cylindrical mold (130) to form the submicron features.

6. The process of one of the preceding claims, wherein molded submicron surface features comprise macropores having a width larger than 50 nm or wherein the molded submicron surface features comprise nanopores having a width not exceeding 50 nm.

7. The process of any of claims 1 to 5, wherein the ceramic region comprises titanium oxide, zirconium oxide, or iridium oxide.

8. The process of any of claims 1 to 5, wherein the ceramic region comprises a mixture of two or more metal or semi-metal oxides or wherein the ceramic region comprises a metal or semi-metal oxide and a polymer.

9. The process of any of claims 1 to 5, wherein the ceramic region is a ceramic layer disposed over all or over a portion of an underlying medical device substrate (110).

10. The process of claim 9, wherein the substrate (110) is a metallic substrate (110).

11. The process of claim 10,
wherein the substrate (110) comprises a substantially pure metal selected from titanium and platinum, or
wherein the substrate (100) comprises a metal alloy selected from nickeltitanium alloys, stainless steels, platinum enhanced radiopaque stainless steels, and cobalt-chromium-molybdenum alloys, or
wherein the substrate (100) is a biodegradable metallic substrate that comprises a substantially pure metal or a metal alloy selected from alkali metals, alkaline earth metals, iron, zinc, and combinations thereof.

12. The process of any of claims 1 to 5, wherein the medical device (100) is a non-planar medical device, a tubular medical device, or a stent.

13. The process of any of claims 1 to 5,
wherein the medical device (100) is a stent that comprises molded submicron surface features on its inner surface, or
wherein the medical device (100) is a stent that comprises molded submicron surface features on its outer surface.

14. The process of any of claims 1 to 5, wherein said medical device (100) comprises a therapeutic agent.

15. The process of claim 14, wherein the therapeutic agent is disposed within the ceramic region

16. The process of any of claims 1 to 5, wherein the medical device (100) is a stent and wherein the molded submicron surface features comprise linear surface features extending parallel to the axis of the stent upon stent deployment.

## Patentansprüche

1. Verfahren zum Bereitstellen einer implantierbaren oder einführbaren medizinischen Vorrichtung (100) umfassend einen Sol-Gel abgeleiteten keramischen Bereich, der geformte Oberflächenmerkmale im Submikrometerbereich umfasst, wobei das Verfahren die folgenden Schritte beinhaltet:
Aufbringen eines Sol-Gel-Precursors (120) auf eine Form (130) oder ein Substrat (110) für eine medizinische Vorrichtung;
Aufbringen der Form (130) auf das Substrat (110) für die medizinische Vorrichtung nach dem Aufbringen des Sol-Gel-Precursors (120); und
Entfernen der Form (130) nachdem der Sol-Gel-Precursor (120) getrocknet ist.

2. Verfahren nach Anspruch 1, wobei die medizinische Vorrichtung (100) eine röhrenförmige medizinische Vorrichtung ist und wobei:
der Sol-Gel-Precursor (120) auf die Form (130) aufgebracht wird; und
Aufbringen der Form (130) Wickeln der Sol-Gel-Precursor beschichteten Form (130) um das Substrat (110) beinhaltet.

3. Verfahren nach Anspruch 1, wobei die medizinische Vorrichtung (100) eine röhrenförmige medizinische Vorrichtung ist und wobei:
der Sol-Gel-Precursor (120) auf die Form (130) aufgebracht wird; und
Aufbringen der Form (130) Wickeln der Sol-Gel-Precursor beschichteten Form (130) um das Substrat (110) beinhaltet, wobei die Form (130) auf der Innenseite des Substrats (110) positioniert ist.

4. Verfahren nach Anspruch 1, wobei die medizinische Vorrichtung (100) eine röhrenförmige medizinische Vorrichtung ist und die Form (130) eine zylindrische Form (130) ist, wobei
der Sol-Gel-Precursor (120) auf eine hohle innere Oberfläche der medizinischen Vorrichtung aufgebracht ist; und
die zylindrische Form (130) um einen inneren Umfang der Sol-Gel-Precursor beschichteten medizinischen Vorrichtung gerollt wird, um die Merkmale im Submikrometerbereich zu bilden.

5. Verfahren nach Anspruch 1, wobei die medizinische Vorrichtung (100) eine röhrenförmige medizinische Vorrichtung ist und die Form (130) eine zylindrische Form (130) ist, wobei
der Sol-Gel-Precursor (120) auf eine hohle innere Oberfläche der medizinischen Vorrichtung aufgebracht ist; und
das Substrat für die medizinische Vorrichtung (110) um die zylindrische Form (130) gerollt wird, um die Merkmale im Submikrometerbereich zu bilden.

6. Verfahren nach einem der vorangehenden Ansprüche, wobei die geformten Oberflächenmerkmale im Submikrometerbereich Makroporen umfassen mit einer Breite größer als 50 nm oder wobei die geformten Oberflächenmerkmale im Submikrometerbereich Nanoporen haben mit einer Breite, die 50 nm nicht überschreitet.

7. Verfahren nach irgendeinem der Ansprüche 1 bis 5, wobei der keramische Bereich Titanoxid, Zirkonoxid oder Iridiumoxid umfasst.

8. Verfahren nach irgendeinem der Ansprüche 1 bis 5, wobei der keramische Bereich eine Mixtur aus zwei oder mehreren Metall- oder Halbmetalloxiden umfasst oder wobei der keramische Bereich ein Metalloder Halbmetalloxid und ein Polymer umfasst.

9. Verfahren nach irgendeinem der Ansprüche 1 bis 5, wobei der keramische Bereich eine keramische Schicht ist, angeordnet über dem gesamten oder über einem Teil von einem zugrunde liegenden Substrat für eine medizinische Vorrichtung (110).

10. Verfahren nach Anspruch 9, wobei das Substrat (110) ein metallisches Substrat (110) ist.

11. Verfahren nach Anspruch 10,
wobei das Substrat (110) ein im Wesentlichen reines Metall umfasst, ausgewählt aus Titan und Platin, oder
wobei das Substrat (110) eine Metalllegierung umfasst, ausgewählt aus Nickel-Titan-Legierungen, Edelstahl, mit Platin angereichertem röntgenopakem Edelstahl und Kobalt-Chrom-Molybdän-Legierungen, oder wobei das Substrat (110) ein biodegradierbares metallisches Substrat ist, das im Wesentlichen reines Metall oder eine Metalllegierung umfasst, ausgewählt aus Alkalimetallen, Erdalkalimetallen, Eisen, Zink und Kombinationen davon.

12. Verfahren nach irgendeinem der Ansprüche 1 bis 5, wobei die medizinische Vorrichtung (100) eine nicht planare medizinische Vorrichtung, eine röhrenförmige medizinische Vorrichtung oder ein Stent ist.

13. Verfahren nach irgendeinem der Ansprüche 1 bis 5,
wobei die medizinische Vorrichtung (100) ein Stent ist, der geformte Oberflächenmerkmale im Submikrometerbereich auf seiner inneren Oberfläche umfasst, oder
wobei die medizinische Vorrichtung (100) eine Stent ist, der geformte Oberflächenmerkmale im Submikrometerbereich auf seiner äußeren Oberfläche umfasst.

14. Verfahren nach irgendeinem der Ansprüche 1 bis 5, wobei die medizinische Vorrichtung (100) einen therapeutischen Wirkstoff umfasst.

15. Verfahren nach Anspruch 14, wobei der therapeutische Wirkstoff innerhalb des keramischen Bereichs angeordnet ist.

16. Verfahren nach irgendeinem der Ansprüche 1 bis 5, wobei die medizinische Vorrichtung (100) ein Stent ist und wobei die geformten Oberflächenmerkmale im Submikrometerbereich lineare Oberflächenmerkmale umfassen, die sich parallel zur Achse des Stents nach der Stent-Einführung erstrecken.

## Revendications

1. Procédé pour fabriquer un dispositif médical implantable ou insérable (100) comportant une région en céramique dérivée de sol-gel qui présente des caractéristiques de surface moulées de l'ordre du sous-micron, comprenant les étapes suivantes:
appliquer un précurseur de sol-gel (120) à un moule (130) ou à un substrat (110) de dispositif médical;
appliquer le moule (130) au substrat (110) de dispositif médical après l'application du précurseur de sol-gel (120); et
retirer le moule (130) après que le précurseur de sol-gel (120) soit séché.

2. Procédé selon la revendication 1, dans lequel le dispositif médical (100) est un dispositif médical tubulaire, et dans lequel:
on applique le précurseur de sol-gel (120) au moule (130); et
l'application du moule (130) inclut l'enroulement du moule (130) revêtu de précurseur de sol-gel autour du substrat (110).

3. Procédé selon la revendication 1, dans lequel le dispositif médical (100) est un dispositif médical tubulaire, et dans lequel:
on applique le précurseur de sol-gel (120) au moule (130); et
l'application du moule (130) inclut l'enroulement du moule (130) revêtu de précurseur de sol-gel autour du substrat (110), dans lequel le moule (130) est positionné sur le côté intérieur du substrat (110).

4. Procédé selon la revendication 1, dans lequel le dispositif médical (100) est un dispositif médical tubulaire et le moule (130) est un moule cylindrique (130), dans lequel le précurseur de sol-gel (120) est appliqué sur une surface intérieure creuse du dispositif médical; et
le moule cylindrique (130) est enroulé autour d'une circonférence intérieure du dispositif médical revêtu du précurseur de sol-gel afin de former les caractéristiques de l'ordre du sous-micron.

5. Procédé selon la revendication 1, dans lequel le dispositif médical (100) est un dispositif médical tubulaire et le moule (130) est un moule cylindrique (130), dans lequel le précurseur de sol-gel (120) est appliqué sur une surface intérieure creuse du dispositif médical; et
le substrat (110) du dispositif médical est enroulé autour du moule cylindrique (130) afin de former les caractéristiques de l'ordre du sous-micron.

6. Procédé selon l'une quelconque des revendications précédentes, dans lequel les caractéristiques de surface moulées de l'ordre du sous-micron comprennent des macropores qui présentent une largeur supérieure à 50 nm ou dans lequel les caractéristiques de surface moulées de l'ordre du sous-micron comprennent des nanopores qui présentent une largeur ne dépassant pas 50 nm.

7. Procédé selon l'une quelconque des revendications 1 à 5, dans lequel la région en céramique comprend de l'oxyde de titane, de l'oxyde de zirconium, ou de l'oxyde d'iridium.

8. Procédé selon l'une quelconque des revendications 1 à 5, dans lequel la région en céramique comprend un mélange de deux ou plus de deux oxydes métalliques ou semi-métalliques ou dans lequel la région en céramique comprend un oxyde métallique ou semi-métallique et un polymère.

9. Procédé selon l'une quelconque des revendications 1 à 5, dans lequel la région en céramique est une couche de céramique déposée sur la totalité ou sur une partie d'un substrat de dispositif médical sous-jacent (110).

10. Procédé selon la revendication 9, dans lequel le substrat (110) est un substrat métallique (110).

11. Procédé selon la revendication 10, dans lequel le substrat (110) comprend un métal sensiblement pur sélectionné parmi le titane et le platine, ou dans lequel le substrat (110) comprend un alliage métallique sélectionné parmi les alliages nickel-titane, les aciers inoxydables, les aciers inoxydables radio-opaques renforcés au platine, et les alliages cobalt-chromemolybdène, ou dans lequel le substrat (110) est un substrat métallique biodégradable qui comprend un métal sensiblement pur ou un alliage métallique sélectionné parmi les métaux alcalins, les métaux alcalinoterreux, le fer, le zinc, et des combinaisons de ceux-ci.

12. Procédé selon l'une quelconque des revendications 1 à 5, dans lequel le dispositif médical (100) est un dispositif médical non plan, un dispositif médical tubulaire, ou un stent.

13. Procédé selon l'une quelconque des revendications 1 à 5, dans lequel le dispositif médical (100) est un stent qui présente des caractéristiques de surface moulées de l'ordre du sous-micron sur sa surface intérieure, ou dans lequel le dispositif médical (100) est un stent qui présente des caractéristiques de surface moulées de l'ordre du sous-micron sur sa surface extérieure.

14. Procédé selon l'une quelconque des revendications 1 à 5, dans lequel ledit dispositif médical (100) comprend un agent thérapeutique.

15. Procédé selon la revendication 14, dans lequel l'agent thérapeutique est disposé à l'intérieur de la région en céramique.

16. Procédé selon l'une quelconque des revendications 1 à 5, dans lequel le dispositif médical (100) est un stent et dans lequel les caractéristiques de surface moulées de l'ordre du sous-micron comprennent des caractéristiques de surface linéaires qui s'étendent parallèlement à l'axe du stent lors du déploiement du stent.
